# EUROPEAN PATENT SPECIFICATION

(11) **EP 0 702 976 B1**
(45) Date of publication and mention of the grant of the patent: **16.04.2003**
(21) Application number: 95202535.1
(22) Date of filing: 19.09.1995
(51) Int. Cl.: A61M 25/01

(54) **MR-visible catheter with elements of paramagnetic material**
MR-sichtbarer Katheter mit Elementen aus paramagnetischem Material
Cathéter, visible par Imagerie par résonance magnetique, avec des éléments en matériau paramagnétique

(30) Priority: 21.09.1994 NL 9401533
(43) Date of publication of application: 27.03.1996
(73) Proprietor: CORDIS EUROPA N.V., NL-9301 LJ Roden (NL)
(72) Inventor: Weber, Jan, NL-9302 ER Roden (NL); van Erp, Wilhelmus Petrus Martinus Maria, NL-9351 KS Leek (NL)
(74) Representative: 't Jong, Bastiaan Jacob

(56) References cited:
- EP-A- 0 537 895
- EP-A- 0 701 835
- EP-A- 0 701 836
- WO-A-94/15534
- US-A- 4 764 321
- US-A- 5 154 179

## Description

The invention relates to a catheter comprising a tubelike body with a proximal and a distal end and with at least one lumen, in which at least one element made of paramagnetic material has been received.

Such a catheter is known from US-A-5 154 179. Such a catheter is furthermore known from documents EP-A-0 701 835 and EP-A-0 701 836. These documents however fall under the provisions of Article 54(3) EPC.

In this known catheter, the element made of paramagnetic material is formed by a liquid or gel contrast medium, received in separate channel in the catheter. Therefore, the known catheter can only be made with a substantial diameter.

The object of the invention is to provide a catheter of the type as described before, that can be produced with a small diameter.

According to the invention, this object is achieved with the catheter as characterized in claim 1.

By using at least some of the wires of the reenforcing layer as elements made of paramagnetic material, a small diameter catheter can be manufactured in usual manner.

The additional advantage is obtained that the visibility of the catheter is ensured irrespective of the direction of the magnetic field.

By choosing the number of wires made paramagnetic material in a suitable manner, a correct degree of visibility of the catheter can be achieved. When a weak paramagnetic material is used, many or all wires can be made of that material. With stronger paramagnetic material less wires will be made of that material in order to achieve good visibility. Thus a suitable choice can be made considering cost price and strength of the reinforcing layer. The non-magnetic material can be a non-magnetic metal, but also a strong plastic material such as KEVLAR^{(TM)} or TWARON^{(TM)}.

The wires of paramagnetic material can be manufactured in a suitable manner of an alloy of non-magnetic material and strong paramagnetic material. Thus suitable tensile-strength properties and magnetic properties can be combined in the material.

A further developed embodiment of the invention is characterized in claim 4. The same method of construction can in this case be employed as for catheters which are to be made visible when subjected to X-rays. The usual marking rings made of gold or platinum are replaced by rings made of paramagnetic material.

A very advantageous embodiment is characterized in claim 5. This is a multipurpose catheter which can be used in conjunction with both X-rays and NMR techniques in order to make the area of the patient to be treated visible.

A suitable embodiment is characterized in claim 6. A paramagnetic material can be used which as such may be harmful to man and is therefore not allowed to come into contact with parts of the human body or body fluids. As the paramagnetic material is situated on the inside of the ring made of non-magnetic material, such contact is out of the question.

Suitable materials are characterized in claim 9.

Suitable materials are characterised in claim 9.

The invention will be explained in greater detail in the following description with reference to the attached drawings.

Figure 1 shows a partly cut away and broken away side view of part of a catheter with a reinforcing layer braided of metal wire, according to the invention.

Figure 2 shows a corresponding view of another embodiment of the catheter according to the invention.

Figure 3 shows a cross-section along the line III-III of figure 2.

Figure 4 shows a partly broken away view of an end-section of a catheter according to another embodiment which is not part of the invention.

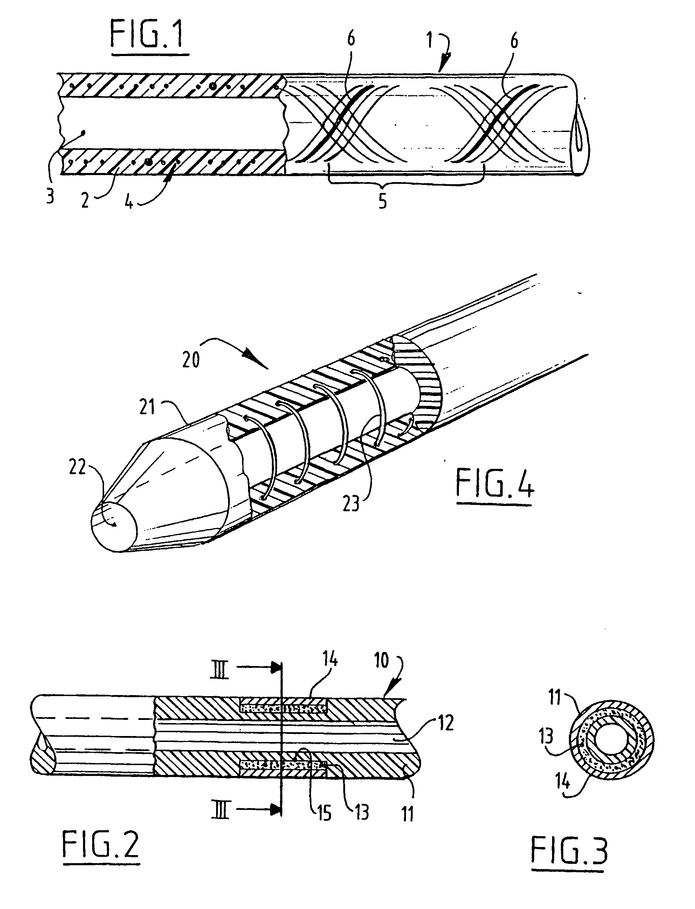

The catheter 1 shown in figure 1 comprises in the usual manner a tube-like body 2 with a lumen 3. The catheter 1 is of the type comprising a reinforcing layer 4 braided of metal wire.

With this catheter one of the wires of which the reinforcing layer 4 has been braided, the one indicated with the number 6, has been made of paramagnetic material. The other wires 5 have been made of a non-magnetic material.

The wires 5 of non-magnetic material remain invisible under NMR conditions, whereas wire 6 made of paramagnetic material causes just enough disturbance of the uniformity of the magnetic field, to render the catheter visible on the screen.

The wire 6 has been made of an alloy of non-magnetic material and strong paramagnetic material, more in particular an alloy of titanium and copper or nickel.

With other embodiments more than one or even all wires may have been made of paramagnetic material.

The catheter 10 of figure 2 also comprises a tube-like basic body 11 with a lumen 12. The body 11 comprises a plastic material commonly used for this purpose, such as for instance polyethene or polyurethane.

In the body 11 a depression 15 has been arranged in which two concentric rings 13, 14 have been received. The ring 13 has been made of paramagnetic material and the ring 14 of non-magnetic material. The rings 13 and 14 have been glued in the depression 15, so that they make up a whole with the basic body 11. The ring 13 made of paramagnetic material renders the catheter visible under NMR conditions, whereas the ring 14 made of non-magnetic material such as platinum or gold renders the catheter visible when subjected to X-rays. The catheter 10 can therefore be used universally, independent of the imaging technique employed.

With the illustrated embodiment of the catheter according to the invention, the paramagnetic material of which the ring 13 has been made can as such be harmful to man. As the ring 13 has been received fully enclosed both by the body 11 of the catheter and the ring 14, no direct contact between the body or body fluids and the paramagnetic material 13 can take place. The paramagnetic material of which the ring 13 is to be made can therefore be chosen freely in accordance with the optimum properties as regards the intended imaging under NMR conditions.

With another embodiment of the invention the ring of paramagnetic material can be made by depositing the paramagnetic material on the inside surface of a ring made of non-magnetic material by means of a deposition technique. Preferably, this deposition technique can be a sputter technique. Thus one single, easy to handle ring is obtained, simplifying the manufacturing process of the catheter.

With the catheter 20 of figure 4 another solution (which does not form part of the invention) is shown for receiving an element of paramagnetic material in the catheter. With the catheter 20 this element is formed by a helically wound wire 23 which is embedded in the plastic material of the body 21 of the catheter 20.

Both on the outside and on the side of the lumen 22, the wire 23 is screened off by material of which the body 20 has been made, so that there is no objection to choose as paramagnetic material for the wire 23, material which has optimal properties where image formation under NMR conditions is concerned but which as such may be harmful to man.

By winding the wire in a helical fashion, it does not effect the flexibility of the end-section of the catheter 20.

## Claims

1. Catheter (1) comprising a tube-like body (2) with a proximal and a distal end and with at least one lumen (3), in which at least one element made of paramagnetic material has been received, **characterized in that** said tube-like body (2) comprises a braided reinforcing layer (4) and the element of paramagnetic material is formed by at least some of the wires (6) of the reinforcing layer (4), which extend helically with regard to the tube-like body (2), and any remaining wires (5) of the reinforcing layer have been made of non-magnetic material.

2. Catheter as claimed in claim 1, wherein the wires of paramagnetic material have been made of an alloy of non-magnetic material and strong paramagnetic material.

3. Catheter as claimed in claim 2, wherein the alloy comprises at least one material from the group including titanium, copper and nickel.

4. Catheter as claimed in claim 1, further comprising at least one marking ring of paramagnetic material arranged around the catheter.

5. Catheter as claimed in claim 4, comprising an assembly of two concentric marking rings made of a non-magnetic material visible when subjected to X-rays, and of a paramagnetic material respectively.

6. Catheter as claimed in claim 5, wherein the ring of paramagnetic material has been formed by means of a deposition technique on the internal surface of the ring made of non-magnetic material.

7. Catheter as claimed in claim 6, wherein the deposition technique is a sputter technique.

8. Catheter as claimed in one of the claims 4-6, wherein the non-magnetic material visible when subjected to X-rays, comprises at least one material from the group including gold and platinum.

9. Catheter as claimed in one of the previous claims, wherein the paramagnetic material has been chosen from the group comprising transition metals such as copper, manganese, chromium, nickel, gadolinium, dysprosium and mixtures, alloys and salts thereof.

## Patentansprüche

1. Katheter (1), umfassend einen röhrenförmigen Körper (2) mit einem proximalen und einem distalen Ende und mit zumindest einem Lumen (3), in das zumindest ein aus paramagnetischem Material hergestelltes Element eingebracht ist, **dadurch gekennzeichnet, dass** der röhrenförmige Körper (2) eine geflochtene Verstärkungsschicht (4) umfasst und das Element aus paramagnetischem Material durch zumindest einige der Drähte (6) der Verstärkungsschicht (4) gebildet ist, welche sich wendeiförmig in Bezug auf den röhrenförmigen Körper (2) erstrecken, und jegliche verbleibenden Drähte (5) der Verstärkungsschicht aus nicht-magnetischem Material hergestellt sind.

2. Katheter, wie in Anspruch 1 beansprucht, wobei die Drähte aus paramagnetischem Material aus einer Legierung von nicht-magnetischem Material und starkem paramagnetischen Material hergestellt sind.

3. Katheter, wie in Anspruch 2 beansprucht, wobei die Legierung zumindest ein Material aus der Gruppe umfasst, die Titan, Kupfer und Nickel enthält.

4. Katheter, wie in Anspruch 1 beansprucht, weiterhin umfassend zumindest einen Markierungsring aus paramagnetischem Material, der um den Katheter herum angeordnet ist.

5. Katheter, wie in Anspruch 4 beansprucht, umfassend eine Anordnung von zwei konzentrischen Markierungsringen, die aus nicht-magnetischem Material, das sichtbar ist, wenn es Röntgenstrahlen ausgesetzt ist, bzw. aus einem paramagnetischen Material hergestellt sind.

6. Katheter, wie in Anspruch 5 beansprucht, wobei der Ring aus paramagnetischem Material mittels einer Abscheidungstechnik auf der inneren Oberfläche des aus nicht-magnetischem Material gemachten Rings gebildet worden ist.

7. Katheter, wie in Anspruch 6 beansprucht, wobei die Abscheidungstechnik eine Zerstäubungstechnik ist.

8. Katheter, wie in einem der Ansprüche 4 bis 6 beansprucht, wobei das nicht-magnetische Material, das sichtbar ist, wenn es Röntgenstrahlen ausgesetzt ist, zumindest ein Material aus der Gruppe umfasst, die Gold und Platin enthält.

9. Katheter, wie in einem der vorstehenden Ansprüche beansprucht, wobei das paramagnetische Material aus der Gruppe ausgewählt worden ist, die Übergangsmetalle, wie Kupfer, Mangan, Chrom, Nickel, Gadolinium, Dysprosium und Mischungen, Legierungen und Salze davon umfasst.

## Revendications

1. Cathéter (1) comprenant un corps en forme de tube (2) avec une extrémité proximale et une extrémité distale et avec au moins une lumière (3), dans laquelle est reçu au moins un élément composé d'un matériau paramagnétique, **caractérisé en ce que** le corps en forme de tube (2) comprend une couche tressée de renforcement (4) et l'élément de matériau paramagnétique est composé d'au moins quelques-uns des fils métalliques (6) de la couche de renforcement (4), qui s'étend de manière hélicoïdale en ce qui concerne le corps en forme de tube (2), et les autres fils métalliques restants (5) de la couche de renforcement ont été composés à partir d'un matériau non magnétique.

2. Cathéter selon la revendication 1, dans lequel les fils métalliques du matériau paramagnétique sont composés d'un alliage de matériau non magnétique et de matériau paramagnétique résistant.

3. Cathéter selon la revendication 2, dans lequel l'alliage comprend au moins un matériau du groupe comprenant le titane, le cuivre et le nickel.

4. Cathéter selon la revendication 1, comprenant en outre au moins un anneau de marquage en matériau paramagnétique agencé autour du cathéter.

5. Cathéter selon la revendication 4, comprenant un assemblage de deux anneaux de marquage concentriques composés d'un matériau non magnétique visible quand il est soumis aux rayons X, et d'un matériau paramagnétique respectivement.

6. Cathéter selon la revendication 5, dans lequel l'anneau de matériau paramagnétique a été composé au moyen d'une technique de dépôt sur la surface interne de l'anneau composé à partir d'un matériau non magnétique.

7. Cathéter selon la revendication 6, dans lequel la technique de dépôt est une technique de pulvérisation.

8. Cathéter selon l'une quelconque des revendications 4 à 6, dans lequel le matériau non magnétique visible quand il est soumis aux rayons X, comprend au moins un matériau du groupe comprenant l'or et le platine.

9. Cathéter selon l'une quelconque des revendications précédentes, dans lequel le matériau paramagnétique a été choisi dans le groupe comprenant des métaux de transition tels que le cuivre, le manganèse, le chrome, le nickel, le gadolinium, le dysprosium et des mélanges, alliages et sels de ceux-ci.
